# EUROPEAN PATENT APPLICATION

(11) **EP 4 406 876 A1**
(43) Date of publication of application: **31.07.2024**
(21) Application number: 23153662.4
(22) Date of filing: 27.01.2023
(51) Int. Cl.: B65D 75/32, B65B 11/52, B65B 11/50, B01L 3/00, B29C 65/58, B29C 65/00

(54) **METHOD OF MANUFACTURING RAPID TEST DEVICES AND A RAPID TEST DEVICE**

(71) Applicant: Feral GmbH, 10997 Berlin (DE)
(72) Inventor: von Hauswolff, Filip, 10405 Berlin (DE); Kössler, Franziska, 81679 München (DE); La Manna, Fabio, 10437 Berlin (DE); Hosseinizad, Seyedehtara, 10587 Berlin (DE); Rapti, Eirini, 10405 Berlin (DE); Adornetto, Gianluca, 10997 Berlin (DE)
(74) Representative: Gulde & Partner

(57) **Abstract**

The present disclosure refers to a rapid test device (1000) and a method of manufacturing rapid test devices (1000) for detecting a target analyte in a fluid sample, the method comprising the steps of: providing (S100) a top member (10) of a thermoformable material including a plurality of identically formed cavities (12) separated from each other in a driving direction (D), wherein the cavities (12) are embossed by thermoforming of the top member (10) and include at least one opening (14) in the cavities (12). The method further comprises the step of driving (S200) the top member (10) in the driving direction (D) by a driving unit (20). The method further includes the steps of inserting (S300) lateral flow test strips (34) into corresponding thermoformed cavities (12) of the top member (10). In a further step, the method includes the step of coupling (S400) a bottom member (60) to the top member (10) covering the cavities (12) to enclose the lateral flow test strips (34). Further, the method includes the step of cutting (S500) at separation lines between adjacent cavities (12) to obtain individual rapid test devices (1000; 1000A, 1000B, 1000C, 1000D, 1000E).

## Description

### Field of the Disclosure

The present disclosure relates to a method of manufacturing rapid test devices for detecting a target analyte in a fluid sample and a corresponding system which performs the method. Further, a corresponding rapid test device is provided which can be obtained by the manufacturing process.

### Technological Background

Rapid test devices generally include lateral flow assays, LFA, or in other words lateral flow strips which are capable of detecting a target analyte without substantial lab equipment.

Rapid test devices are commonly operated in hospitals or clinical laboratories for qualitative detection of specific antigens in different fluid samples such as blood, saliva or urine. There is further a growing demand for using rapid test devices in home-based environments. Example target analytes reach from detecting fertility hormones, antigens of corona or flu viruses but are not restricted thereto. Other examples can also include different types of biological matter dissolved in buffer as sample type.

The active ingredient of a rapid test device is the LFA which typically includes a plurality of pads coupled to each other. A sample fluid can be transported along the pads causing the sample fluid to perform selected biochemical reactions eventually indicating a positive or negative test result related to the presence or absence of the target analyte.

The LFA strip is typically enclosed in a plastic cassette which are obtained by injection moulding according to known manufacturing processes. These cassettes are manufactured by injection moulding including rigid injection moulded top and bottom enclosing an LFA strip in the cassette.

Such manufacturing process is not only complex but also consumes large amounts of plastics. Since rapid test devices are disposable, i.e., only used once, this causes extensive plastic waste which is further amplified due to the increased use of home-based testing. In addition, substantial investment costs are needed for the manufacturing.

Therefore, there is a need to provide an improved manufacturing process for rapid test devices and rapid test devices which involve less amount of plastic in the manufacturing process and as well in the final product. In addition to the above, the production scheme needs improved efficiency and must be suitable for large scale production. Further problems are solved as will be indicated in the below concrete description.

### Summary of invention

The invention is defined by the appended claims. The description that follows is subjected to this limitation. Any disclosure lying outside the scope of said claims is only intended for illustrative as well as comparative purposes.

A method of manufacturing rapid test devices for detecting a target analyte in a fluid sample is disclosed. The method comprises the steps of providing a top member of a thermoformable material including a plurality of identically formed cavities separated from each other in a driving direction, wherein the cavities are embossed by thermoforming of the top member and include at least one opening. The method further comprises the step of driving the top member in the driving direction by a driving unit. The method further includes the steps of inserting lateral flow test strips into corresponding thermoformed cavities of the top member. In a further step, the method includes the step of coupling a bottom member to the top member covering the cavities to enclose the lateral flow test strips. Further, the method includes the step of cutting at separation lines between adjacent cavities to obtain individual rapid test devices.

Further, a rapid test device for detecting a target analyte in a fluid sample. A thermoformed top portion including at least one opening is provided. The thermoformed top portion is coupled with the bottom portion coupled to form a housing. A lateral flow test strip is accommodated in the housing.

Further aspects of the present disclosure could be learned from the dependent claims or the following description.

### Brief Description of the Drawings

Features will become apparent to those of ordinary skill in the art by describing in detail exemplary embodiments with reference to the attached drawings in which:
- Fig. 1: illustrates a schematic representation of a method of manufacturing rapid test devices for detecting a target analyte in a fluid sample according to embodiments of the invention.
- Fig. 2: illustrates a method of manufacturing rapid test devices for detecting a target analyte in a fluid sample according to a first embodiment of the invention.
- Fig. 3: illustrates a method of picking and placing fluid test strips into the thermoplastic cavities according to an embodiment.
- Fig. 4: illustrates a method of providing a top member according to embodiments of the invention.
- Fig. 5: illustrates a method of manufacturing rapid test devices for detecting a target analyte in a fluid sample according to a second embodiment of the invention.
- Fig. 6: illustrates a method of manufacturing rapid test devices for detecting a target analyte in a fluid sample according to a third embodiment of the invention.
- Fig. 7: illustrates rapid test devices according to embodiments of the invention.
- Fig. 8: illustrates a method of coupling a bottom member to a top member according to an embodiment of the invention by showing cross sections at different steps.
- Fig 9: illustrates a method of manufacturing a rapid test device according to another embodiment of the invention.

### Detailed Description of the invention

Reference will now be made in detail to embodiments, examples of which are illustrated in the accompanying drawings. Effects and features of the exemplary embodiments, and implementation methods thereof will be described with reference to the accompanying drawings. In the drawings, like reference numerals denote like elements, and redundant descriptions are omitted. The present disclosure, however, may be embodied in various different forms, and should not be construed as being limited to only the illustrated embodiments herein. Rather, these embodiments are provided as examples so that this disclosure will be thorough and complete, and will fully convey the aspects and features of the present disclosure to those skilled in the art. In the drawings, the relative sizes of elements, layers, and regions may be exaggerated for clarity.

As used herein, the term "and/or" includes any and all combinations of one or more of the associated listed items. Further, the use of "may" when describing embodiments of the present disclosure refers to "one or more embodiments of the present disclosure.". Expressions such as "at least one of," when preceding a list of elements, modify the entire list of elements and do not modify the individual elements of the list.

It will be further understood that the terms "include," "comprise," "including," or "comprising" specify a property, a region, a fixed number, a step, a process, an element, a component, and a combination thereof but do not exclude other properties, regions, fixed numbers, steps, processes, elements, components, and combinations thereof.

In the drawings, the sizes of elements may be exaggerated for clarity. For example, in the drawings, the size or thickness of each element may be arbitrarily shown for illustrative purposes, and thus the embodiments of the present disclosure should not be construed as being limited thereto.

Unless otherwise defined, all terms (including technical and scientific terms) used herein have the same meaning as commonly understood by one of ordinary skill in the art to which the present disclosure belongs. It will be further understood that terms, such as those defined in commonly used dictionaries, should be interpreted as having a meaning that is consistent with their meaning in the context of the relevant art and/or the present specification, and should not be interpreted in an idealized or overly formal sense, unless expressly so defined herein.

### General Concept

According to one aspect of the present disclosure, method of manufacturing rapid test devices for detecting a target analyte is provided. In addition, a corresponding rapid test device is provided according to the present disclosure.

The method comprises the steps of providing a top member of a thermoformable material including a plurality of identically formed cavities disposed or arranged along a line in a driving direction. The cavities are embossed by thermoforming of the top member and include at least one opening in each of the cavities. The method further comprises the step of moving the top member in the driving direction by a driving unit as for example a motor. The method further includes the steps of inserting lateral flow test strips into corresponding thermoformed cavities of the top member, i.e., in each cavity a corresponding lateral flow test strip. In a further step, the method includes the step of connecting a bottom member to the top member covering the cavities for enclosing the lateral flow test strips. Further, the method includes the step of cutting, after coupling the top member with the bottom member, at separation lines between adjacent cavities to obtain individual rapid test devices.

The rapid test device for detecting a target analyte in a fluid sample includes a thermoformed top portion having at least one opening. A bottom portion is coupled to the thermoformed top portion to form a common housing. A lateral flow test strip is accommodated, i.e., inserted, in the housing.

The separation line may be a line perpendicular to the driving direction between adjacent cavities. The thermoformable material is a material which can be subject to a thermoforming process. A driving unit may be a motor, for example an electric motor, but the invention is not restricted thereto. The cutting may be provided by a slitter, a cutter or a punching die, for example. The insertion operation may be provided by a pick and placing unit including, for example, a robot, sorter or more general a device that is capable of moving and inserting the lateral flow strips into the formed cavities within the top member, for example a robot. In addition, the process of inserting may be done by a human operator filling the lateral flow strips, which provides flexibility having various degrees of automation. A cavity may be in other words an accommodation space for accommodating a lateral flow test strip. The top member may be in other words an elongate sheet member. The top member is formed to be suitable for a manufacturing line. The top member may be fed in reel-form or as individual sheets depending on the scale of the manufacturing line. At least one control unit may control the above manufacturing steps to obtain the rapid test device. The bottom member may be a sticker. The bottom member may be made from a non-polymer bio-based material, for example a wax-coated paper or similar with adequate waterproof properties may be used. According to an embodiment, the thermoformable material may be a thermoplastic material, for example, a blister material. The top member may include at least one among PLA, PET, PLA, rPET, PS etc.

The described method has the advantage that a fast manufacturing process is provided which allows producing large amounts of rapid test devices while having reduced plastic usage compared to conventional injection molded manufacturing processes. In particular, due to the use of a thermoformable top member, the amount of plastic for the rapid test devices can be reduced by an order of magnitude. That is, an amount of 70-90% of plastic can be saved compared to conventional injection molded plastic cassettes. Thus, natural environment pollution can be substantially reduced, in particular, when considering the rising demand on rapid test devices and considering that these devices are usually disposable.

The advantages in a direct comparison with respect to the manufacturing method versus traditional injection moulded plastic manufacturing process include the following. Firstly, costs are drastically reduced since less material needs to be purchased and processed. Secondly, environmental impact is reduced since less materials means less embodied energy per test. Thirdly, the plastic content is vastly reduced not only by a thinner top thermoformed part but also the bottom member, in particular, when it is made from a non-polymer bio-based material or a wax-coated paper or similar with adequate waterproof properties may be used. In a further advantage, reduced costs of tooling of thermoforming tools versus injection moulding tools and automated assembly system as a whole are achieved. Most of the processes in traditional rapid test assembly deal with orienting the parts with efficient feeding (bulk etc) uniformly for simple manipulations.

According to an embodiment, the thermoformable material may be a thermoplastic or a thermoformable paper. Compared to injection moulding, the thermoformed top portion when using thermoplastics can be made very thin and so plastic amount reduced. In particular, fiber-based materials may be used. In case thermoformable paper is used, no plastics may be needed so that the above-described effects are maximized. Further, both materials can be provided from reel or sheet thermoformed to provide a disposable housing yet with adequate features specific to lateral flow tests.

According to an embodiment, the thermoformed cavities include one or more retention cleats, and the step of inserting of the individual flow test strips into the respective cavities includes inserting the lateral flow test strips between the one or more retention cleats. The retention cleats may be provided to facilitate the function of aligning. The retention cleats may include opposing nobs. The retention cleats are also obtained by the thermoforming and thus can be provided together with forming the cavities.

According to an embodiment, the bottom member may be flat or even, and the coupling of the bottom member includes unrolling the bottom member from a bottom member reel and couple the bottom member to the top member through an adhesive to enclose the lateral flow test strips. When the bottom member is fed from a reel the orientation is already known and set. Thus, the feeding is vastly simplified in comparison to feeding loose cassette plastics in a hopper or bowl feeder. The adhesive may have an additional advantage to seal the bottom of the lateral flow strip itself.

According to an embodiment, the coupling of the bottom member may include mechanically coupling of the bottom member to the top member by a mechanical press-fit to enclose the lateral flow test strips. Here, each of the top member and the bottom member may include coupling profiles which allow a snapping together. The coupling profiles, i.e., the side profiles, may have a profile surface that extends in the same direction with respect to each other so that one coupling profiles can be seated into the other coupling profile. The coupling profiles may be either both inwardly indented or both outwardly protruding. The coupling may be referred to as a mating detent. The process may include the coupling profile of the bottom member to flex outward and pressed down until the coupling profile is seated in the coupling profile of the top member, i.e., forming a mating detent feature. The bottom member and the top member may both include coupling profiles which may be both produced by thermoforming. For example, the coupling profiles may be in-line formed or provided with the pre-formed cavities. Again, the mating detent may also be provided by coupling profiles both protruding outwardly.

According to embodiments, the coupling profiles may extend along the side. Thus, they provide additional rigidity. The top member and the bottom member may better support each other structurally and become more robust despite of the thin material of thermoplastic or thermoformable paper. Thus, there would be no need for an adhesive bonding in such coupling. In addition, the bottom member may carry further features required for some rapid test devices such as a 'raised' bed to float the later flow strip from the bottom surface.

According to an embodiment, the inserting of the lateral flow test strips may include the steps of: Providing a lateral flow strip member including a plurality of identically formed lateral flow test strips. In a further step the method may include the step of cutting, by a first cutter or a first slitter, the lateral flow strip member at separation lines between adjacent lateral flow test strips to obtain individual flow test strips. In addition, the method may include inserting the cut individual lateral flow test strips into the corresponding thermoformed cavities of the top member. The lateral flow strip member may be a card member or a reel-form member. The inserting may be performed by a pick and place unit like a robot.

For example, if a slitter is used it may slit/cut a card member or reel-form member into lateral flow strips at once or simultaneously. This may be a rotary cutter through which the card member is passed, in illustrative words, like a pasta machine. In an example, a 30 cm card member would equal 75 strips at once if each strip is 4 mm wide. In such case, the cut lateral flow test strips would need to be arranged and spaced on a stage for a pick and place unit to take, for example, 5/10/20 at a time and place within each incrementing cycle given by the prior thermoforming process in the system. For another example, if a strip cutter is used each cut would yield one separated lateral flow strip. However, the cutter may operate at a high speed and thus is useful in a few different scenarios. For example, in a continuous motion system, i.e., where rotary in-line thermoforming is performed as explained further below, there would be no stop between thermoforming cycles. Here, a (high-speed) cutter may suffice and instead rely on a higher speed of a pick and place unit to follow the rate of feeding of top member and cavities. Furthermore, similar to the slitter, a (high-speed) cutter can cut strips which are deposited onto a conveyor with specific spacing, i.e., a stage from which again, multiples of 5/10 cut lateral flow strips could be taken with each pick and place into the cavities following the cycle of the thermoforming process.

In another embodiment, the step of inserting of the lateral flow test strips includes cutting at a plurality of separation lines to obtain a plurality of test strips in a cutting cycle and simultaneously inserting the plurality of cut lateral flow test strips into corresponding thermoformed cavities of the top member while stopping the driving of the top member in the driving direction.

Thus, for each cutting cycle and insertion step, the production rate of the manufacturing process can be increased by a factor corresponding to the number of simultaneously placed lateral flows test strips. Thus, scalability is provided in this process step which importantly allows for high production rates and production efficiency.

Stopping may additionally allow to ensure correct positioning and/or fixation of the cut lateral flows test strips in the cavities. Due to the above-described scalability, during the stoppage time, instead of only one lateral test strip, a number of lateral flow test strips is inserted so that the stoppage time is more efficiently used. Further, for a given amount of lateral flow test strips, the total stoppage time is reduced. This implies a higher production rate. Further, in case of inline thermoforming, as explained further below, a stoppage time may be equal to a cycle time for the thermoforming operation to take place before incrementing the top member.

According to an embodiment, the lateral flow test strip member is positioned adjacent to the top member in the driving direction, and the inserting of the plurality of flow test strips into the corresponding thermoformed cavities of the top member includes a planar shifting of the plurality of cut lateral flow test strips perpendicular to the driving direction before inserting the lateral flow test strips. Thus, by the said positioning, due to the parallel and laterally overlapping positioning, a plurality of lateral strips can be readily inserted in a simultaneous manner into corresponding cavities.

According to an embodiment, the cavities are open in an upper direction of the top member, the inserting of the individual flow test strips into the cavities is performed from an upper side of the top member and the lateral flow test strips are placed upside-down into the cavities. To provide this, a pick and place unit may operate a flipping operation to bring the lateral flow test strips in the upside-down position. For example, an intermediate gripper, belt or other manipulator for flipping may be integrated or provided. In other embodiments, the cutting may be provided already by upside down cutting. The cavity thus forms a bottom holder during the manufacturing such that the lateral flow test strips are supported by the bottom holder. For further ensuring of that the lateral test strip stays in place until the bottom member is applied, the lateral flow strip may be attached with an adhesive bond to the top member during insertion. An additional fixation may also be achieved with a vacuum (negative airflow) below the cavities which would hold the lateral flow test strip down until the bottom member is applied.

According to an embodiment, the providing of the top member may include unrolling the top member from a first reel, wherein the top member is pre-formed to include the plurality of identically formed cavities embossed by thermoforming of the top member. Thus, embossing and punching is performed offline in this embodiment, i.e., in the process of preparing the top member. The top member being pre-formed may be an embossed carrier tape which is essentially a member which has the thermoformed cavities and is punched for the at least one opening, and then spun up on a reel to be used later, in particular in a packaging or filling step. Further, when the top member is fed from a reel the orientation is already known and set. Thus, the feeding is vastly simplified in comparison to feeding loose cassette plastics in a hopper or bowl feeder.

This reduces the manufacturing time at the production location since the embossing and punching is already done before so that the amount manufacturing devices and manufacturing steps at the production location is reduced. This may also lead to that an actual failure rate at the production location is reduced.

According to an embodiment, the providing of the top member may include: Unrolling a top member from a reel, and thermoforming of unrolled sections of the top member unrolled from the reel to emboss the plurality of identically formed cavities of the top member. The method further comprises punching, with a punching tool, the at least one opening into the cavities after the step of embossing the cavities by thermoforming. Thus, an inline forming of the cavities is provided by starting the process with an unstructured top member that is raw or in other words even or unstructured. Therefore, the autonomous time can be increased since the method is independent of pre-production and unstructured top members are highly available and can be stored in vast amounts.

According to an embodiment, the thermoforming may include a process of vacuum forming, by a vacuum forming tool, or by applying pressurized air applied to emboss the plurality of identically formed cavities in the unstructured top member. Vacuum forming is a fast and direct way of embossing cavities with desired shape on the top member. The vacuum forming tool includes a heater to locally heat the unrolled section of the top member and a vacuum generator, e.g., a pump, wherein the vacuum forces the top member against a mold that has the desired shape as a negative. Therefore, both vacuum (negative airflow) and pressurized air (positive airflow) can be used to set the top member into a thermoform tool cavity. In the process of thermoforming, the driving of the top member may be temporarily stopped until the embossing is terminated. More accurate cavity structures may be generated in this manner.

According to an embodiment, the thermoforming may include a process of applying a heated rotary forming tool to the top member, the heated rotary forming tool including a structured surface to emboss the plurality of identically formed cavities on the top member. Such form of embossing by a heated rotary forming tool, i.e., a heated roller, allows that the embossing can be performed without stopping the driving of the top member. Thus, a more continuous driving of the top member may be maintained. The process of embossing the top member may thus be more continuous.

According to an embodiment, the providing of a reel-form member may include a plurality of identically formed lateral flow test strips separated from each other includes driving the reel-form member from a third reel. By using a reel compared to a card, an improved autonomous time may be achieved since the reel allows to store larger amounts of lateral flow strips.

According to an embodiment, the thermoformed top member may include one among polylactide, PLA, polystyrene, PS, and polyethylene terephthalate, PET or polyethylene PE. Also, thermoformable paper may be used to entirely avoid plastics. These materials may be suitable allow the embossing either inline or offline to produce the corresponding cavities by thermoforming and to then perform the opening generation.

According to an embodiment, the thickness is between 0.1 to 0.6 mm, preferably between 0.15 mm to 0.5 mm, more preferably between 0.2 mm to 0.3 mm. Therefore, the amount of plastic is reduced while still providing sufficient material strength and rigidity for a single use product.

According to an embodiment, the coupling of the bottom member may include unrolling the bottom member from a bottom member reel and couple the bottom member with the top member through an adhesive to provide the sealed top member. The adhesive may be for example an acrylic pressure-sensitive adhesive, PSA. Thus, an adhesive has the advantage of sealing of the bottom of the lateral flow test strip against leaking capillary draw. Further, the bottom member may be water-proof.

According to an embodiment, also the bottom member may be embossed by thermoforming. For example, the bottom member may include features for aligning and/or retaining the lateral flow strip, if it can further help alignment of parts and for raising the lateral flow strip relative to a bottom surface with the aim of breaking capillary draw between the lateral flow test strip backing card and the bottom member, for example, in the event an adhesive cannot be used.

According to an embodiment, the coupling of the bottom member may include unrolling the bottom member from a bottom member reel and couple the bottom member with the top member on the second side by welding to provide the sealed top member. This may provide a higher autonomous time and may allow coupling during driving of the top member. In such case, compared to adhesive, cost-efficient raw materials for the bottom member could be used. Further, this has an additional advantage for rapid test devices that do not require the adhesive to seal against the bottom of the strip itself.

According to an embodiment, the method may include an edge trimming, wherein the edge trimming is performed together with the cutting, by the second cutter, the sealed top member, or before placing, by the pick and place unit, the cut individual flow test strips into a corresponding thermoformed cavity of the top member. The edge trimming may be performed by a punch die to generate round edges. The edge trimming may reduce a risk for the user of getting injured due to sharp edges. Performing the edge trimming in the final step of cutting may have the advantage that an outer buffer is kept during the manufacturing process, for example, in order to have something for grippers, rollers or other mechanical feature to help advance the cavities and top member through the assembly steps.

In a further aspect of the invention, a system for manufacturing rapid test devices is configured to implement the above manufacturing steps. The system may be in other words a machine. At least one control unit may be provided to operate the various units of the system according to the manufacturing process. The same advantages of the method also apply to the system or machine in other words.

In a further aspect of the invention, a rapid test device obtained by the manufacturing process according to the above embodiments is provided. By using the thermoforming of the thermoformable top member can reduce the amount of plastic for the rapid test devices by an order of magnitude, i.e., an amount of 70-90% of plastic can be saved compared to conventional plastic cassettes. Thus, substantial natural environment pollution may be reduced.

In a further aspect of the invention, a rapid test device for detecting a target analyte in a fluid sample is provided. The rapid test device includes a thermoformed top portion including at least one opening. A bottom portion is coupled to the top portion to obtain a housing. A lateral flow test strip accommodated in the housing. The rapid test device may have the same additional features as described above according to various embodiments which are not repeated for the sake conciseness. The advantages correspond to the advantages as presented above with respect to the manufacturing process.

The thermoformed top portion may be of a thermoplastic material or of a thermoformable paper.

The thermoformed top portion may include retention cleats, and the individual flow test strip is positioned between the one or more retention cleats.

The bottom portion may be flat, and the bottom portion may be coupled to the thermoformed top portion through an adhesive to enclose the lateral flow test strip.

The bottom portion may be flat, and the bottom portion may be coupled to the thermoformed top portion through welding to enclose the lateral flow test strip.

The bottom portion may be mechanically coupled to the thermoformed top portion by a mechanical press-fit to enclose the lateral flow test strip.

In an embodiment, a thickness of the thermoformed top portion may be between 0.1 to 0.6 mm, preferably between 0.15 mm to 0.5 mm, more preferably between 0.2 mm to 0.3 mm.

In an embodiment, the bottom portion may be thermoformed. For example, the bottom portion may include (additional) retention cleats for aligning and/or retaining the lateral flow test strip. The above-described features regarding the rapid test device can be combined together.

In a further aspect of the invention, a manufacturing of an above-described rapid test device for detecting a target analyte in a fluid sample includes the following steps. In a first step, the method may include providing a thermoformed top portion including at least one opening. In a further step the process includes providing a lateral flow strip. In a further step, the process includes the step of coupling a bottom portion to the top portion to form a housing and to enclose the lateral flow test strip in the housing. The method may have the same additional features as described above and in the entire application according to the various embodiments which are not repeated for the sake conciseness. The method as described above has the advantage that such assembly can be done at a location avoiding a large-scale production scheme. For example, such process can be performed when the bottom portion, lateral flow test strip and thermoformed top portion are individually provided and then assembled. The various forms of coupling of the bottom portion, the retention cleats and/or the material selection and thicknesses and the various other embodiments described in this application directly translate to the above manufacturing process and can be combined therewith.

### Specific Embodiments

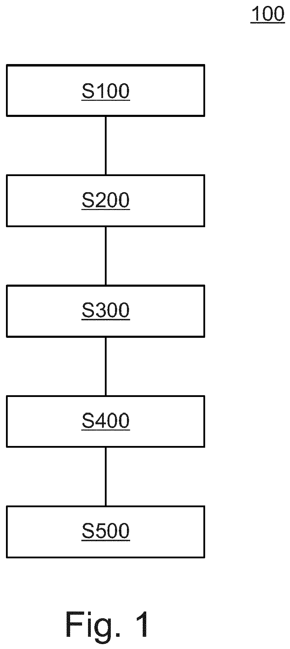

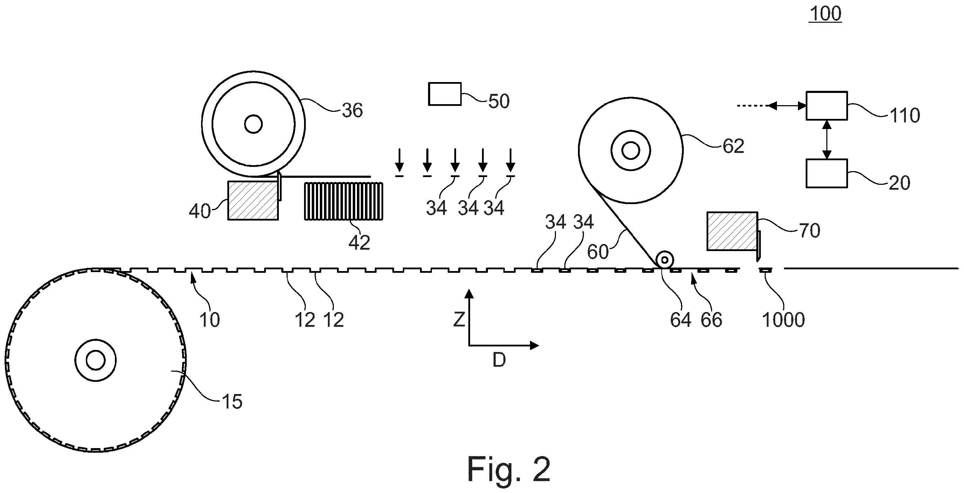

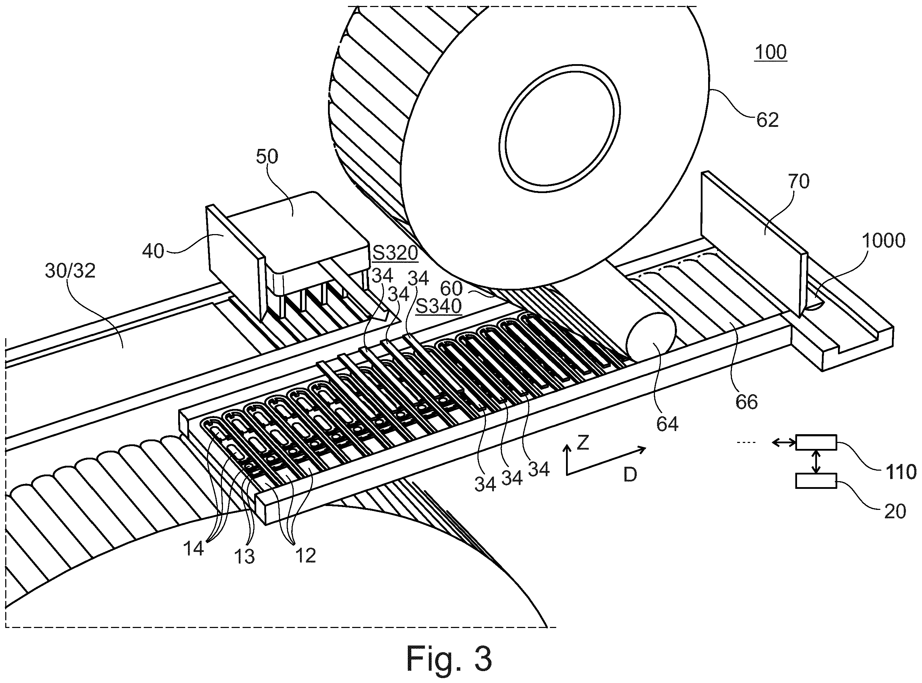

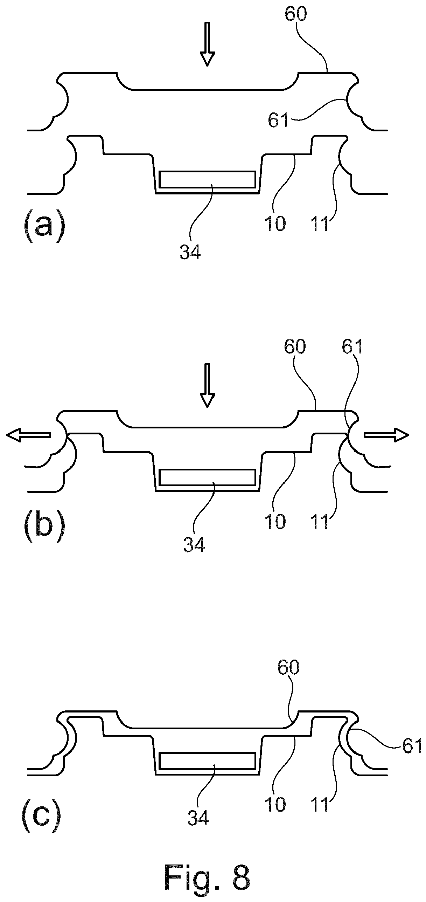

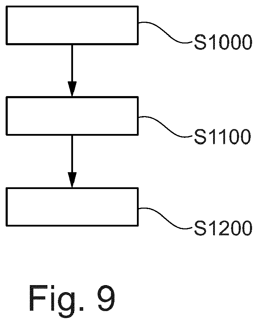

Fig. 1 illustrates a schematic representation of a method and a system 100 for manufacturing rapid test devices 1000. The rapid test devices 1000 are capable of detecting a target analyte in a fluid sample according to various embodiments of the invention. The method will be described and illustrated in the following by referring to the embodiment of Fig. 2 and the various embodiments as disclosed in Figs. 3-6 in the following. Reference is also made to the specific mechanical coupling as provided in Figs. 8 and 9. Fig. 2 illustrates a method of manufacturing rapid test devices for detecting a target analyte in a fluid sample according to a first embodiment of the invention which will be described in the following together with Fig. 1.

According to Fig. 1, a method of manufacturing rapid test devices 1000 for detecting a target analyte in a fluid sample is disclosed. The method can be executed by a system 100 or machine for manufacturing rapid test devices 1000. The system 100 may comprise the components described in the following in the context of Figs. 1 to 2 and also the embodiments as presented in Figs. 3 to 6 and 8 to perform the steps as described below. In particular, at least one control unit 110 is provided to operate the system 100 and to control the various components of the system 100 to perform the manufacturing steps.

The manufacturing method according the embodiment of Fig. 1 includes providing S100 a top member 10 of a thermoformable material. In particular, a thermoplastic material for the top member 10 or a thermoformable paper may be used as described further above. The thermoplastic materials may include one among polylactide, PLA, polystyrene, PS, and polyethylene terephthalate, PET or polyethylene PE, but are not limited thereto. For example, the material may include also bio-based PE, rPET or others etc. All these materials can advantageously be subject to thermoforming as used in the present invention.

To reduce an amount of plastic while maintaining stiffness and mechanical strength, a thickness of the top member 10 may be in the intervals of 0.1 to 0.6 mm, preferably in intervals of 0.15 mm to 0.4 mm, more preferably between 0.2 mm to 0.3 mm. The material selection, wherein each combination of thickness and material selection is disclosed, as well applies to the other embodiments as described with respect to the embodiments of the present invention.

The top member 10 includes a plurality of cavities 12, which are for example illustrated in Fig. 2 or Fig. 3. The cavities 12 are separated from each other on the top member 10 regarding a driving direction D as illustrated in Fig. 2. The driving direction D is the direction in which the top member 10, i.e. the manufacturing line, is moved or driven to be exposed to the manufacturing steps for eventually obtaining the rapid test devices 1000.

In the example embodiment as shown in Fig. 2, the top member 10 is provided S100 by unrolling the top member 10 from a top member reel 15. Thus, in this case, the top member 10 is pre-formed to include the plurality of identically formed cavities 12. In other words, in this embodiment, the cavities 12 are generated offline and provided to the system 100 or production location to be used for the manufacturing process. The cavities 12 are embossed by thermoforming of the top member 10. Thus, the production location and the manufacturing system 100 is less complex since the embossing through thermoforming does not have to be performed during the manufacturing process but is performed offline.

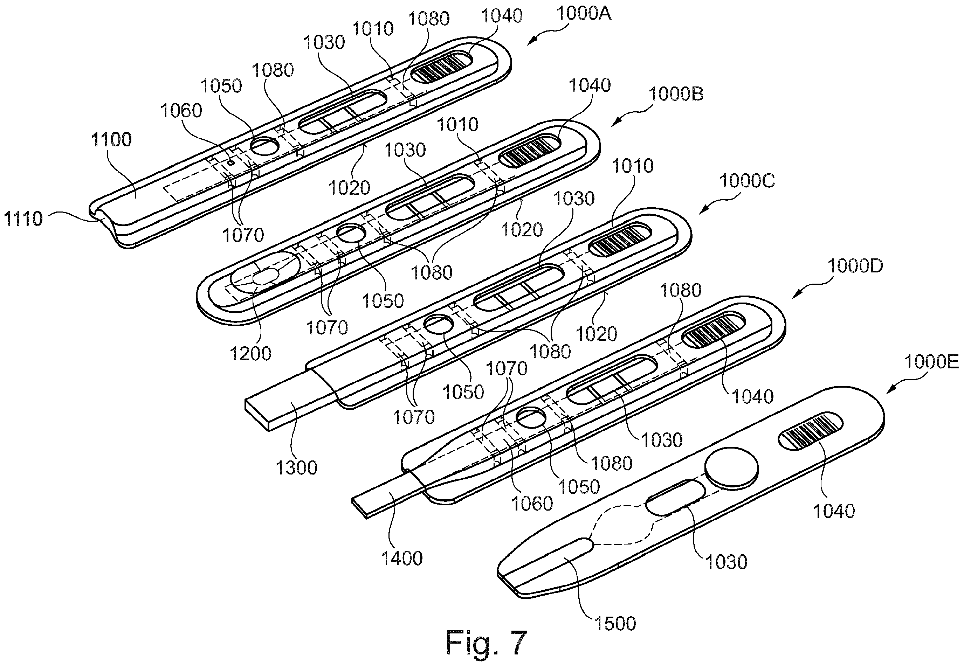

The cavities 12 of the top member 10 are embossed, here offline, by thermoforming of the top member 10. In this way, the thermoformable property, i.e., thermoplastic or thermoformable paper, is used to generate desired cavities 12, for example having desired shape and depth through the thermoforming treatment. The cavities 12 further include at least one opening 14 in each of the cavities 12. The openings 14 may be windows indicating visual feedback to the user or to provide an overflow protection feature that allows excess sample fluid to exit for to ensure the integrity of the testing. For example, different types of openings 14 are illustrated in Fig. 7 and at least one of them may be provided.

The top member 10 is driven S200, i.e., moved, by a driver unit 20, for example a motor, in the driving direction D which is for example illustrated in Figs. 2 and 3. A rail or conveyor belt may be provided to facilitate the motion of the top member 10 in the driving direction D. The driving operation may be controlled by the at least control unit 110 of the system 100.

The method further includes the step of inserting S300 lateral flow test strips 34 into corresponding thermoformed cavities 12 of the top member 10. That means that each of the thermoformed cavities 12 may receive a corresponding lateral flow test strip 34.

The step of inserting S300 may include providing a lateral flow strip member 30, 32, for example as a card-form member 30 or a reel-form member 32. The card-form member 30 or the reel-form member 32 may include a plurality of identically formed lateral flow test strips 34. Thus, the card-form member 30 and the reel-form member 32 are pre-formed offline. Preferably, a card-form member 30 or a reel-form member 32 is positioned or moved in the driving direction D parallel to the top member 10. This is illustrated in Fig. 2 and more particularly in Fig. 3. The motion may be in synchronization with the driving of the top member 10 in the driving direction D.

When the reel-form member 32 is used, the providing S300 of the reel-form member 32 may include unrolling the reel-form member 32 from a lateral flow test strip reel 36 as illustrated in Fig. 2. In this way, when compared to using a card-form member 30, an autonomous time of the production process may be enhanced.

The method further includes a step of cutting of the lateral flow strip member 30, 32. This process may be performed by a first cutter 40 or a first slitter 42 as illustrated in Fig. 2. The lateral flow strip member 30, 32 may be cut at separation lines between adjacent lateral flow test strips 34 to obtain individual lateral flow test strips 34. This is for example schematically illustrated in Fig. 2 or more explicitly in Fig. 3. In this way, a high degree of automation can be reached. The separation lines are lines perpendicular to the driving direction D thus allowing to disconnect the lateral flow test strips 34 from each other. Cutting or slitting materials are provided by the cutter and slitter to ease the process of cutting according to whether card-form member 32 or reel-form member 3234 is used.

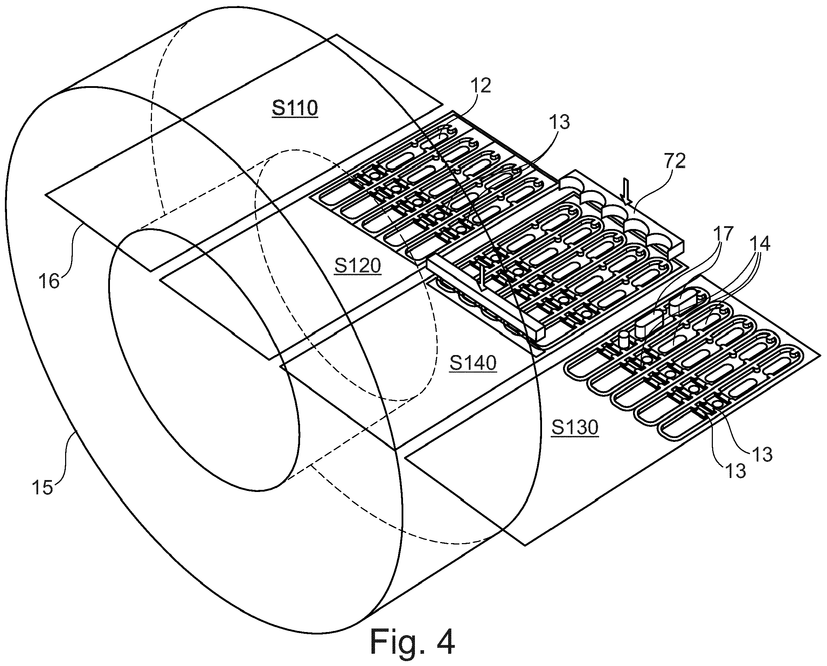

The cut individual flow test strips 34 may be inserted into corresponding thermoformed cavities 12 of the top member 10. Thus, the cavities 12 are used to accommodate the lateral flow test strips 34. Therefore, the flow test strips 34 are positioned along the driving direction D in the top member 10 as indicated in Figs. 2 and 3. A corresponding pick and place unit 50 may be used to provide the insertion function of the cut individual lateral flow test strips 34. The operation of the pick and place unit 50 may be synchronized with the driving of the top member 10 so that the flow test strips 34 can be inserted in the provided cavities 12. For example, a temporary stopping of the driving of the top member 10 may be provided to make the insertion process more accurate. To facilitate the step of inserting the lateral flow test strips 34, the thermoformed cavities 12 may include one or more retention cleats 13. Preferably, a plurality, here three as example, retention cleats 13 may be provided. Then, the step of inserting S300 of the lateral flow test strips 34 into the respective cavities 12 includes inserting and positioning the lateral flow test strips 34 by the one or more retention cleats 13. Thus, the function of aligning is improved. The retention cleats 13 may include opposing nobs. The retention cleats 13 can be also obtained by the thermoforming. The cleats 13 thus help to secure align the lateral flow test strip 34 so that they are stably inserted and held in the cavities 12. The cleats 13 are illustrated in Fig. 3 and also in Fig. 4.

In particular, since the cavities 12 are extended in a bottom direction (-z- direction) when being driven in driving direction D, the inserting S300 of the individual flow test strips 34 into the cavities 12 is performed upside-down into the cavities 12. In this way, the cavity 12 supports the flow test strips 34 through gravitation support during the manufacturing and during the motion. The pick and place unit 50 may include a flipping operation to bring the lateral flow test strips 34 in the upside-down position for insertion. For example, an intermediate gripper, belt or other manipulator for flipping may be provided or integrated. Alternatively, upside down cutting may be provided.

In a further operating step, a bottom member 60 is coupled S400 with the thermoformed top member 10. The bottom member 60 is coupled with the top member 10 to provide a sealed top member 66, i.e., in which the lateral flow test strip 34 is enclosed or surrounded. This step is illustrated in Fig. 2 or Fig. 3. After this step, the inserted lateral flow test strips 34 are entirely surrounded or sealed by the bottom member 60 and the thermoformed top member 10. In other words, the lateral flow test strips 34 are housed in the top member 10 together with the bottom member 60.

The coupling S400 of the bottom member 60 may include unrolling the bottom member 60 from a bottom member reel 62, as illustrated in Fig. 2 or Fig. 3. The bottom member 60 can then be coupled with the top member 10 through an adhesive to provide the sealed top member 66 and to enclose the lateral flow test strips 34. An auxiliary reel 64 may be provided to support the coupling process of the bottom member 60. In this manner, enhanced autonomous times may be provided due to the reel solution. The adhesive can provide an additional sealing effect for the lateral flow test strip 34. In further embodiments, the bottom member 60 can be coupled with the top member 10 through welding to enclose the lateral flow test strips 34. In this manner, a sealed top member 66 is provided. For example, heat welding or laser welding may be used for the laser coupling process.

In yet another embodiment, reference is made to Fig. 8 which illustrate that the coupling S400 of the bottom member 60 can include mechanically coupling of the bottom member 60 to the top member 10 by a mechanical press-fit to enclose the lateral flow test strip 34. Here, each of the top member 10 and the bottom member 60 may include a coupling profiles 11, 61 which allow snapping together. The coupling profiles 11, 61 extend or are curved in the same direction with respect each other so that a coupling profile can be seated into the other coupling profile. In the present case the coupling profiles 11, 61 are both (inwardly) indented as shown in Fig. 8 part (a). In another embodiment not shown, the coupling profiles 11, 61 may be both (outwardly) protruding. The coupling may be referred to as a mating detent. In the present case, the coupling profile 61 of the bottom member 60 flexes outward, as seen in Fig. 8 (b) with respect to Fig. 8 (a), and it is pressed down until the coupling profile 61 is seated in the coupling profile 11 of the top member 10 as shown in Fig. 8 (c). At least an upper edge of the coupling profile 11 mechanically prevents decoupling of the bottom member 60. Here, the bottom member 60 and the top member 10 may both include coupling profiles 11, 61 which may be produced in the thermoforming process. For example, the coupling profiles 11, 61 may be in-line formed or provided with the pre-formed cavities. Again, the mating detent may also be provided by coupling profiles 11, 61 both extending outwardly.

Since the coupling profiles 11, 61 may extend along the side, they can provide additional rigidity to the device. The top member 10 and the bottom member 60 may better support each other structurally and become more robust despite of the thin material of thermoplastic or thermoformable paper. Thus, there would be no need for an adhesive bonding. In addition, the bottom member 60 may carry further features required for some rapid test devices such as a 'raised' bed to float the later flow strip 34 from the bottom surface. The above-described method of mechanically coupling may be applied as well in the context of the manufacturing method of Fig. 9 by referring to the bottom portion and the thermoformed portion.

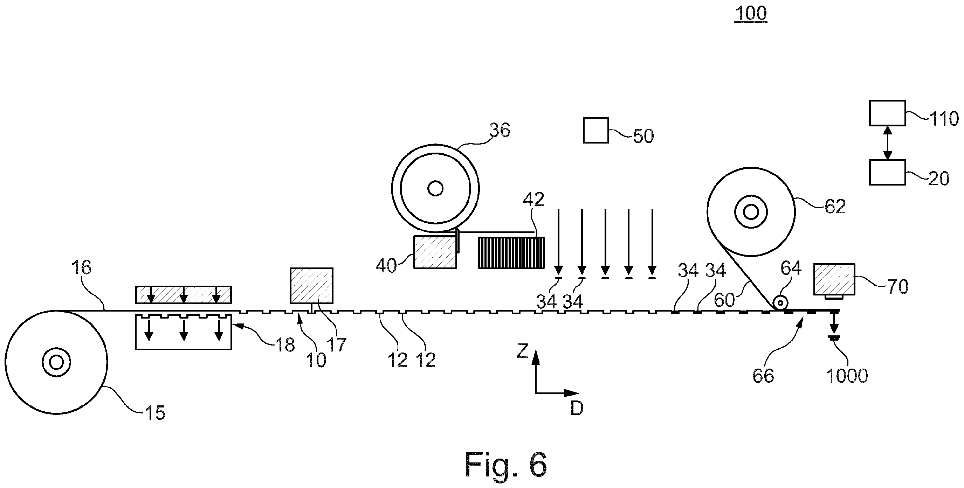

The method further comprises a step of cutting S500 the sealed top member 10 at separation lines between adjacent cavities 12. For example, a second cutter 70 or a second slitter may be used. In alternative, also a punching die could be used as illustrated in Fig. 6. This would enable further forming of the part (in case the edges have not yet been trimmed round in prior steps) or if a bigger 'buffer' zone between the cavities 12 is required instead of a zero-waste-margin as illustrated in here. The separation lines may be lines perpendicular to the driving direction D to separate the cavities 12 from each other. Due to the cutting, individual rapid test devices 1000; 1000A, 1000B, 1000C, 1000D, 1000E are obtained in the final state. The rapid test devices 1000; 1000A, 1000B, 1000C, 1000D, 1000E are then outputted as shown in Figs. 2 and 3. The outputted rapid test devices may be collected and stored for transport or packaging. In a further step, the method may include an edge trimming. The edge trimming may be performed together with the cutting S500 of the sealed top member 66. In this manner, undesired sharp corners or edges can be rounded so that a risk of injury is reduced. This is for example illustrated in Fig. 6, but can also be applied in the various other embodiments.

In the above-described manner, a method of manufacturing rapid test devices 1000 for detecting a target analyte in a fluid sample is provided which is fast and reduces the amount of plastic during the process as well as for the final product due to the use of the thermoformed top member 10, e.g., when compared with injection molded cassettes according to the prior art as already described above in more detail. In particular, autonomous times can be enhanced by specific embodiments thereof and the manufacturing tools reduced.

In Fig. 3 a preferred method of inserting S400 lateral flow test strips 34 into the thermoformed cavities 12 according to an embodiment is described which allows scalability so that production rates can be increased as will be described below.

A first cutter 40 or a first slitter 42 can be used to cut the lateral flow strip member 30, 32, card-form member 30 or the reel-form member 32, at separation lines between adjacent lateral flow test strips 34 to obtain individual flow test strips 34 which is illustrated in Fig. 3.

In this preferred embodiment, a cutting S320 is performed at a plurality of successive or adjacent separation lines to obtain at least two flow test strips 34 in a cutting cycle. For example, a simultaneous cutting at the plurality of separation lines is performed for being most time efficient. In alternative, a sequence of cuttings is performed to obtain the plurality of individual flow test strips 34.

Once the plurality of flow test strips 34 is provided, the method includes a simultaneous inserting S3400 of the plurality of flow test strips 34 into corresponding thermoformed cavities 12 of the top member 10. Thus, in one cutting cycle, a plurality of insertions of flow test strips 34 is reached. In this manner, a scalable feature is implemented in the manufacturing process. This increases the production rates and reduces the processing time for a given target number of rapid test devices. Therefore, efficiency of the entire production process is achieved. That is, a multiplied insertion reduces overall speed of the system. In motion of the robotics, motors etc. involved one can make 10 strips in a cycle instead of 10 single placements at a very high speed. The higher the speed of the assembly, the more robust placements and tolerances etc need to be to avoid parts ending up in the wrong position etc.

The process of cutting and the process of inserting may be synchronized by the at least one control unit 110 of the system 100.

The method may include to stop the driving S200 of the top member 12 in the driving direction D when inserting the plurality of flow test strips 34 into the corresponding thermoformed cavities 12 of the top member 10. This allows to securely place and insert the plurality of lateral flow test strips 34 into the cavities 12 in simultaneous manner, for example, as described above to achieve the press-fit engagement. The stoppage time or waiting time, however, is drastically reduced due to that in each stoppage window a plurality of lateral flow test strips 34 can be inserted. Thus, production rates can be increased to facilitate large scale production. In a further improvement, the stopping may be synchronized with the cycle time of the thermoforming step, when performed in-line as described further below with respect to Figs. 4 to 6. For example, if there are 20 cavities in the tool and a cycle takes 5 seconds, the stopping would be for 5 seconds to allow transfer and placement (inserting) of the lateral flow test strips 34. Of course, a longer cycle time can also be achieved if the inserting, e.g., the picking and placing, requires so. In such embodiment, the time loss due to the in-line thermoforming operation as relevant source of speed reduction may be optimized in synchrony, as in each thermoforming cycle required a heating of the top member 10, applying pressurized air or vacuum to make the sheet material conform to the tool and open the tool, allow for cooling, i.e., for example crystallization of the polymer or colling of paper before incrementing the web as described below in the context of Figs. 4 to 6.

In this particular embodiment, the lateral flow strip member 30, 32, for example the card-form member 30 or reel-form member 32, is positioned adjacent to the top member 10. In other words, they extend parallel to each other in the driving direction D. Thus, by this relative geometry an overlap section in the driving direction D is provided as illustrated in Fig. 3. This can be used as described in the following. The placing S500 of the plurality of flow test strips 34 into the corresponding thermoformed cavities 12 of the top member 10 may be performed by a planar shifting of the cut lateral flow test strips 34 perpendicular to the driving direction D across the transition. This setting is very suitable for the simultaneous placing of many lateral flow test strips 34 into the cavities 12 as illustrated in Fig. 3, where in this example, five lateral flow test strips 34 are placed into the cavities 12 simultaneously by a lateral motion and then being pushed down into the cavities 12 to for example align in the cavity 12, for example using the retention cleats 13, as described above. This allows also free visual access to observe the process and a planar geometry of the system 100 or machine with additional benefits for in-line vision inspection system (camera and reject identifier). That is, in case a lateral flow test strip 34 is misplaced or missing the system 100 could be configured to scrap the lateral flow test strip 34. This also may be beneficial for both bad part rejection but also to help synchronize the top and bottom members 10, 60 in the stage of coupling S400.

Fig. 4 as well as Figs. 5 and 6 illustrate methods of manufacturing rapid test devices for detecting a target analyte in a fluid sample according to other embodiments of the invention.

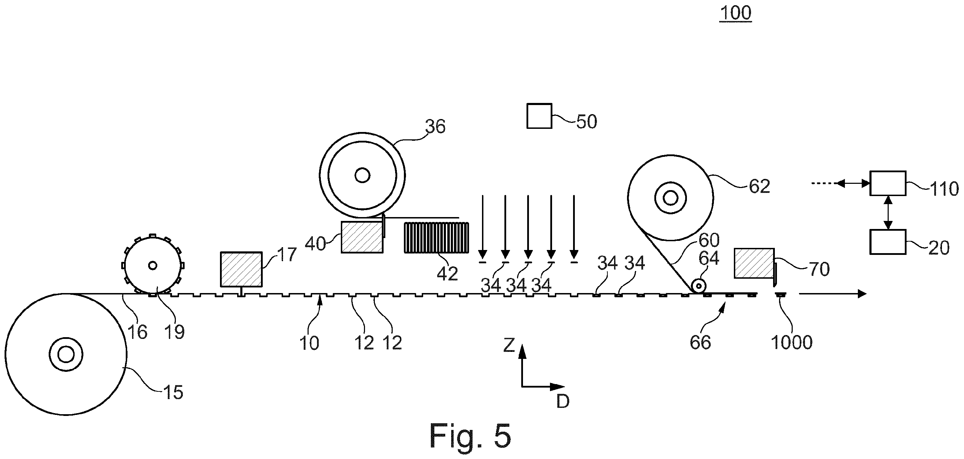

In particular, as illustrated in Fig. 4, a method of providing the top member 10 including the plurality of identically formed cavities 12 is further illustrated and particular embodiments thereof are disclosed with respect to Figs. 5 and 6. In the following, only the differences with respect to Fig. 2 are described, wherein all other features are incorporated herein by reference for the mere sake of conciseness.

As illustrated in Figs. 4 - 6, the providing S100 of the top member 10 includes the following inline operations. The method according to this embodiment includes a step of unrolling S110 of an unstructured top member 16 from a top member reel 15. The unstructured top member 16 compared to Fig. 2 is thus raw or even. The material selection and thickness may be similar as described above. In other words, the unstructured top member 16 is not pre-formed which may allow higher autonomous time compared to the pre-formed solution.

Further, the method includes the step of thermoforming S120 of unrolled sections of the unstructured top member 16 unrolled from the top member reel 15 to emboss the plurality of identically formed cavities 12 in the unstructured top member 16. This step is for example disclosed in the Figs. 4. The cavities 12 for example may include the retention cleats 13 to align the lateral flow test strips 34 when inserted therein as described above. Particular examples of the step of thermoforming S120 are explained with respect to Figs. 5 and 6 below.

The method may further comprise a step of punching S130, with a punching tool 17 as for example a punching die, the at least one opening 14 into the cavities 12 after the step of embossing the cavities 12 by thermoforming S120. The punching tool 17 may be a punching die to generate the at least one opening 14. Thus, various functional windows such as a venting hole or feedback windows can be generated.

The inline forming as described above has the advantage of higher autonomous time compared to the pre-formed reel solution due to the larger length and availability of unstructured top members 16. Thus, the production output can be increased when on the scale of large production times. This also results in a drastic reduction in overall cost of goods, since the main raw material conversion step is brought directly in the line, circumventing the need for a third party to form the cavities and the punched openings. Furthermore, the increase in autonomous time reduces the need for stops to fill material and therefore reducing headcount to operate the production line in this regard.

In this embodiment, an optional step of edge trimming S140 may be additionally executed wherein the edge trimming is performed after the thermoforming S120. The edge trimming may be performed through preformed edge forming die 72 as illustrated in Fig. 4. The edge trimming may provide round edges for reducing injury risk.

Fig. 5 illustrates a method of manufacturing rapid test devices for detecting a target analyte in a fluid sample according to a second embodiment of the invention. Fig. 5 discloses a specific embodiment of Fig. 4.

In this particular embodiment the thermoforming S120 includes a process of applying a heated rotary forming tool 19 to the unstructured top member 16. The heated rotary forming tool 19 includes a structured surface to emboss the plurality of identically formed cavities 12 on the unstructured top member 16 by rotation. The structured surface includes the negative of the cavity 12. A heater can be separately provided or integrated. In this solution, for the step of thermoforming S120, the driving of the unstructured top member 16 does not have to be stopped so that faster production rates may be reached. Thus, the thermoforming can be embedded mor continuously.

Fig. 6 illustrates a method of manufacturing rapid test devices for detecting a target analyte in a fluid sample according to a third embodiment of the invention. Fig. 6 discloses a specific embodiment of Fig. 4.

In this particular embodiment, the thermoforming S120 may include a process of vacuum forming, by a vacuum forming tool 18, as shown in the Figure or to apply pressurized air to emboss the plurality of identically formed cavities 12 of the unstructured top member 16. Using a vacuum forming tool 18 is a particular fast and accurate way of embossing the cavities 12 with desired shape on the unstructured top member 16. The vacuum forming tool 18 may include a heater to locally heat the unrolled section of the unstructured top member 16. Further, a vacuum is generated below the unstructured top member 16 so that atmospheric pressure presses the top member 16 against a mold including the desired cavity 12 as a negative. Again, also pressurized air may be used as alternative.

In the process of inline thermoforming according various embodiments, the driving of the top member may be temporarily stopped until the embossing is terminated. However, accurate cavity structures may be generated in this manner. For example, to be synchronized together with the process of inserting S400.

Fig. 7 illustrates several rapid test devices 1000; 1000A, 1000B, 1000C, 1000D. The rapid test devices may be obtained by the manufacturing process according to the above embodiments. The various embodiments demonstrate that the above-described manufacturing process and steps therein can be applied to many different and desired rapid test devices 1000A, 1000B, 1000C, 1000D, 1000E. These embodiments have in common that they have a low or no plastic content and can be manufactured in a fast and efficient manner according to the embodiments as described above. Thus, all embodiments include a thermoformed top portion 1010 and a bottom portion 1020 coupled to the top portion 1010 to form a housing. The coupling may be achieved through adhesive, welding or mechanical coupling as described in the various embodiments as described above. The bottom portion 1020 corresponds to the bottom member of the previous embodiments and the thermoformed top portion 1010 corresponds to the top member of the previous embodiments for a single rapid test device. The lateral flow test strip 34 is inserted therein to be enclosed by the top portion 1010 and the top portion 1020 as described above. In particular, the rapid test devices 1000 may include the one or more retention cleats 13 allowing an improved inserting and aligning of the lateral flow test strips 34 between the one or more retention cleats 13. The thermoformed top portion 1010 includes the at least one opening 14. The bottom portion may also be thermoformed and embossed. For example, the bottom member may include features for aligning and/or retaining the lateral flow test strip. Various other features described in the context of the above embodiments of manufacturing method may be included in the rapid test device.

According to a first embodiment, a rapid test device 1000A is configured for detecting a target analyte in a saliva sample, e.g., for detecting fertility hormones, antigens for covid flu or as well drugs. However, the invention is not restricted thereto since any analyte that can be measured by lateral flow test strips 34 in a saliva sample to be collected directly in mouth.

In this embodiment, the at least one opening 14 may include at least one among the following openings, wherein each combination is disclosed herein. A test readout window 1030 including a test line and/or a control line may be provided. In a further embodiment, a barcode readout window 1040 overlapping a barcode may be provided. In a further embodiment, a flow indication window 1050 may be provided which can indicate that the sample fluid has reacted in the reactive zone of the lateral flow test strip 34. It may also indicate whether sufficient sample fluid has been collected to initiate the flow development in the lateral flow test strip 34.

In a further embodiment, a venting hole 1060 for saliva overflow prevention may be provided through which undesired excess saliva due to overcollection can be exited.

Further, the rapid test device 1000A includes a mouth port 1110 and a sample cavity 1100 to receive and collect saliva directly from the mouth of the user. The saliva is then fed to the lateral flow test stripe from the sample cavity 1100 through a sample pad extending therein. In addition, flow gates 1070 to protect the lateral flow strip from too much unreacted saliva are provided above a sample pad. Further, an inwardly extending rib 1080 may be provided to secure persistent lateral flow across overlapping pads of the lateral flow test strip by facilitating a contact pressure point.

According a second embodiment, a rapid test device 1000B for detecting a target analyte in a saliva sample is disclosed. Only the differences with respect to the first embodiment are described and for common features it is referred to the above disclosure. Instead of using a mouth port and a sample cavity, the at least one opening includes a sample port 1200 on a top of the device to input a fluid sample. Thus, the sample fluid can be, for example, delivered by means of pipette or buffer bottle in the sample port 1200.

According to a third embodiment, a rapid test device 1000C for detecting a target analyte in a fluid sample is disclosed. Only the differences with respect to the first embodiment are described and for common features it is referred to the above disclosure. Instead of using a mouth port and a sample cavity, the manufacturing concept as well covers rapid test devices, wherein the fluid sample is delivered by wicking. In here, a sample wick 1300 is disclosed which provides the wicking function for the sample fluid to be deposited in the sample wick 1300. The sample fluid is then wicked to the reactive zones of the lateral flow test strip 34. Applications are for example urine based fertility tests, e.g., LH, pregnancy or saliva based tests, e.g., covid, drugs, malaria, fertility, cortisol but the invention is not restricted thereto. Such an embodiment would require one additional assembly step involving an insertion to secure a wick pad before the strip is placed and the bottom applied to seal the rapid test device.

According to a fourth embodiment, a rapid test device 1000D for detecting a target analyte in a fluid sample is disclosed. Only the differences with respect to the third embodiment are described and for common features it is referred to the above disclosure. In this particular embodiment, a urination wick 1400 is disclosed which can directly receive a urination sample.

According to a fifth embodiment, a rapid test device 1000E for detecting a target analyte in a fluid sample is disclosed. Only the differences with respect to the first embodiment are described and for common features it is referred to the above disclosure. The rapid test device 1000E is an embodiment for detecting a target analyte in a blood sample. Here, the sample is delivered by means of capillary draw directly from finger prick (blood) or similar low viscosity fluids. Thus, in this example, capillary channel 1500 is provided for transferral of an applied blood sample toward the reactive zones of the lateral flow test strip 34.

According to Figure 9, another manufacturing process is described according to the invention which results as well in a rapid test device according to the above-mentioned embodiments of the various rapid test devices for detecting a target analyte in a fluid sample 1000; 1000A, 1000B, 1000C, 1000D, 1000E. The method may include the step of providing S1000 a thermoformed top portion 1010 including at least one opening 1030, 1040, 1050. The thermoformed top portion 1010 may have the various features as described above in the context of the device description but also in the context of the description of the manufacturing according to the various embodiments as described above. In a further step, the process includes the step of providing S1100 a lateral flow strip 34.

The process includes the step of coupling S1200 a bottom portion 1020 to the top portion 1010 to form a housing and to enclose the lateral flow test strip 34 in the housing. The method may have the same additional features as described above according to various embodiments which are not repeated for the sake conciseness. The method as described here has the additional advantage that this assembly can be done at a location different from a large-scale production scheme. For example, such process can be performed when the bottom portion, lateral flow test strip and thermoformed top portion are individually provided and the process allows for example for a manual assembly.

In particular, the rapid test devices 1000 may include the one or more retention cleats 13 allowing to insert and align the lateral flow test strip 34 between the one or more retention cleats 13. The various forms of coupling of the bottom portion 1020 including welding, adhesion and in particular the mechanical coupling as described above, the retention cleats 13 and/or the material selection and thicknesses and the various other embodiments described in this application directly translate to the above manufacturing process and can be combined therewith and are only not repeated for avoidance of redundancy. In summary, the presented embodiments disclose a rapid test device and method of manufacturing rapid test devices 1000 for detecting a target analyte in a fluid sample and a corresponding rapid test device 1000. The manufacturing is fast, reduces the amount of plastic or even entirely removes plastic which is made feasible by many of the above amendments. In some embodiment, the process has a scalable feature and the autonomous times may be enhanced. Further advantages are provided in the above detailed sections.

### Reference signs

- 100: system for manufacturing rapid test devices
- 110: control unit

- 10: top member
- 11: coupling profile
- 12: cavity
- 13: retention cleat
- 14: opening
- 15: reel
- 16: unstructured top member
- 17: punching tool/die
- 18: vacuum forming tool
- 19: rotary forming tool

- 20: driving unit

- 30: card-form member
- 32: reel-form member
- 34: lateral flow test strip
- 36: strip foil reel

- 40: first cutter
- 42: first slitter

- 50: pick and place unit/robot

- 60: bottom member
- 61: coupling profile
- 62: bottom member reel
- 64: auxiliary reel
- 66: sealed top member

- 70: second cutter
- 72: edge trimming tool/die

- D: driving direction

- S100: providing
- S110: unrolling
- S120: thermoforming
- S130: punching

- S200: driving

- S300: inserting
- S320: cutting a plurality of lateral flow strips
- S340: simultaneous inserting
- S400: coupling
- S500: cutting

- 1000: rapid test device
- 1000A: rapid test device
- 1000B: rapid test device
- 1000C: rapid test device
- 1000D: rapid test device
- 1000E: rapid test device

- 1010: thermoformed top portion
- 1020: bottom portion

- 1030: test readout window
- 1040: barcode readout window
- 1050: flow indication window
- 1060: venting hole

- 1070: flow gate
- 1080: inwardly extending rib
- 1100: sample cavity
- 1110: mouth port

- 1200: sample port

- 1300: sample wick
- 1400: urination wick

- 1500: capillary channel

- S1000: providing thermoformed top portion
- S1100: providing lateral flow strip
- S1200: coupling a bottom portion

## Claims

1. A method of manufacturing rapid test devices (1000; 1000A, 1000B, 1000C, 1000D, 1000E) for detecting a target analyte in a fluid sample, wherein the method comprises the steps:
- providing (S100) a top member (10) of a thermoformable material including a plurality of identically formed cavities (12) separated from each other in a driving direction (D), wherein the cavities (12) are embossed by thermoforming of the top member (10) and include at least one opening (14) in the cavities (12), and
- driving (S200) the top member (10) in the driving direction (D) by a driving unit (20);
- inserting (S300) lateral flow test strips (34) into corresponding thermoformed cavities (12) of the top member (10);
- coupling (S400) a bottom member (60) to the top member (10) covering the cavities (12) to enclose the lateral flow test strips (34); and
- cutting (S500) at separation lines between adjacent cavities (12) to obtain individual rapid test devices (1000; 1000A, 1000B, 1000C, 1000D, 1000E).

2. The method according to claim 1, wherein the thermoformable material is a thermoplastic material.

3. The method according to claim 1, wherein the thermoformable material is a thermoformable paper.

4. The method according to claims 1 to 3, wherein the thermoformed cavities (12) include one or more retention cleats (13), and the step of inserting (S300) of the individual flow test strips (34) into the respective cavities (12) includes inserting the lateral flow test strips (34) between the one or more retention cleats (13).

5. The method of one of the claims 1 to 4, wherein the bottom member (60) is flat, and the coupling (S400) of the bottom member (60) includes unrolling the bottom member (60) from a bottom member reel (62) and couple the bottom member (60) to the top member (10) through an adhesive or through welding to enclose the lateral flow test strips (34).

6. The method of one of the claims 1 to 4, wherein the coupling (S400) of the bottom member (60) includes mechanically coupling of the bottom member (60) to the top member (10) by a mechanical press-fit to enclose the lateral flow test strips (34).

7. The method according to one of the claims 1 to 6, wherein the providing (S100) of the top member (10) includes unrolling the top member (10) from a reel (15), wherein the top member (10) is pre-formed to include the plurality of identically formed cavities (12) embossed by thermoforming on the top member (10).

8. The method according to claims 1 to 6, wherein the providing (S100) of the top member (10) includes:
- unrolling (S110) an unstructured top member (16) from a reel (15),
- thermoforming (S120) of unrolled sections of the unstructured top member (16) unrolled from the reel (15) to emboss the plurality of cavities (12) of the unstructured top member (16); and
- punching (S130), with a punching tool (17), the at least one opening (14) into the cavities (12) after the step of embossing the cavities (12) by thermoforming.

9. The method according to claim 8, wherein the thermoforming (S120) includes a process of vacuum forming, by a vacuum forming tool (18), or by applying pressurized air to emboss the plurality of identically formed cavities (12) of the unstructured top member (16).

10. The method according to claim 8, wherein the thermoforming (S120) includes a process of applying a heated rotary forming tool (19) to the unstructured top member (16), the heated rotary forming tool (19) including a structured surface to emboss the plurality of identically formed cavities (12) of the unstructured top member (16).

11. The method of one of the claims 1 to 10, wherein a thickness of the top member (10, 16) is between 0.1 to 0.6 mm, preferably between 0.15 mm to 0.5 mm, more preferably between 0.2 mm to 0.3 mm.

12. The method of one of the claims 1 to 11, wherein the inserting (S300) of the lateral flow test strips (34) includes:
- providing a lateral flow strip member (30, 32) including a plurality of identically formed lateral flow test strips (34);
- cutting, by a first cutter (40) or a first slitter (42), the lateral flow strip member (30, 32) at separation lines between adjacent lateral flow test strips (34) to obtain individual flow test strips (34); and
- inserting the cut individual lateral flow test strips (34) into the corresponding thermoformed cavities (12) of the top member (10), wherein the inserting (S300) of the lateral flow test strips (34) includes:
- cutting (S320) at a plurality of separation lines to obtain a plurality of test strips (34) in a cutting cycle; and
- simultaneously inserting (S340) the plurality of cut lateral flow test strips (34) into corresponding thermoformed cavities (12) of the top member (10) while stopping the driving of the thermoplastic foil (10) in the driving direction (D).

13. System (100) for manufacturing rapid test devices (1000; 1000A, 1000B, 1000C, 1000D, 1000E), configured to implement the method according to one of the claims 1 to 12.

14. A rapid test device (1000; 1000A, 1000B, 1000C, 1000D, 1000E) for detecting a target analyte in a fluid sample, including:
a thermoformed top portion (1010) including at least one opening (1030, 1040, 1050),
a bottom portion (1020) coupled to the thermoformed top portion (1010) to form a housing, and
a lateral flow test strip (34) accommodated in the housing.

15. A method of manufacturing the rapid test device (1000; 1000A, 1000B, 1000C, 1000D, 1000E) for detecting a target analyte in a fluid sample according to claim 14, including the steps of:
- providing (S1000) a thermoformed top portion (1010) including at least one opening (1030, 1040, 1050),
- provide (S1100) a lateral flow strip (34); and
- coupling (S1200) a bottom portion (1020) to the top portion (1010) to form a housing and to enclose the lateral flow test strip (34) in the housing.
